# EUROPEAN PATENT APPLICATION

(11) **EP 1 655 035 A1**
(43) Date of publication of application: **10.05.2006**
(21) Application number: 04450206.0
(22) Date of filing: 05.11.2004
(51) Int. Cl.: A61K 38/19, A61P 9/10, A61K 48/00

(54) **Treatment of heart failure with CSF-1**

(71) Applicant: Aharinejad, Seyedhossein, 1130 Wien (AT)
(72) Inventor: Aharinejad, Seyedhossein, 1130 Wien (AT)
(74) Representative: Sonn & Partner Patentanwälte

(57) **Abstract**

Described is the use of CSF-1 for the preparation of a pharmaceutical preparation for the treatment of ischemic heart disease and heart failure.

## Description

Heart failure resulting from ischemic heart diseases is a major public health concern; leading to fibrous scar and associated left ventricular (LV) remodeling (1,2). For end-stage heart failure patients, cardiac transplantation is the only accepted treatment that is being offered and then only to a small percentage of patients.

Therefore, approaches that bypass heterologous organ transplantation are highly desired (3).

In addition to stem cell transfer into the ischemic myocardium (4,5) myoblast transplantation is receiving a growing interest as a means of replacing lost cardiomyocytes by exogenously supplied contractile cells. Skeletal myoblasts feature clinically attractive characteristics, including ease of procurement, growth potential in vitro, and strong resistance to ischemia (6). The ability of engrafted skeletal myoblasts to differentiate into myotubes and to improve post infarction cardiac function has been shown in animal studies (7-12). Also recent reports in humans show that autologous skeletal myoblast transplantation might be a feasible procedure for myocardial repair (6,13-17).

One important issue in myoblast transfer into the failing myocardium is the integration of these cells in the scar tissue that results from infarction. The inability of myoblasts to transdifferentiate into cardiomyocytes and to form a syncytium with neighboring cells has been discussed as a possible mechanism for ventricular arrhythmias that might occur following myoblast transfer into the myocardium (6,14,15). Another significant issue in the setting of myoblast transfer is the lack of patent blood vessels in the fibrous tissue within the infarcted myocardium that could result in degeneration of at least a part of transferred myoblasts.

Based on the above reasoning, a need exists for alternative treatment of heart failure, such as ischemia-induced heart failure.

It is an object of the present invention to provide efficient methods for treating heart failure, especially heart failure resulting from myocardial infarction associated with myocardial remodeling.

The present invention therefore provides the use of CSF-1 (Colony Stimulating Factor; also often termed as "Macrophage Colony Stimulating Factor (M-CSF)) for the pharmaceutical preparation for the treatment of ischemic heart diseases or heart failure.

According to the present invention, a suitable CSF-1 expression in the myocardium has to be at least as twofold than the CSF-1 expression level in normal myocardial tissue and has to be maintained for a sufficient period of time in the heart, especially in the close vicinity of or in the diseased area, e.g. in case of ischemic heart failure. In general, it is not critical, how this suitable, enhanced CSF-1 expression is upheld as long as the period of time is sufficient. The CSF-1 overexpression in myocardium should be maintained for at least one day, although preferentially longer overexpression periods of time, e.g. 2-14 days are desired.

The use or method according to the present invention is applicable to a wide variety of heart disorders and specifically suitable for disorders being related to ischemia, especially chronic ischemia. With the present invention, attenuation of myocardial remodeling, enhancement of angiogenesis and significant improvement of cardiac function is enabled. Preferred heart disorders to be treated according to the present invention include various forms of heart failure.

Specifically preferred is the application according to the present invention for ischemic heart diseases, especially ischemia-induced heart failure.

Other preferred heart disorders which may be treated according to the present invention are coronary heart disease associated with myocardial ischemia, non-ischemic forms of cardiomyopathy, such as dilative, hypertrophic, or restrictive cardiomyopathy.

As mentioned above, it is not critical as to how the enhanced expression of CSF-1 is maintained in or at the heart. Therefore, any administration that allows a stable and localised microenvironment with CSF-1 overexpression is suitable according to the present invention. For example, using a pharmaceutical preparation containing a depot form of CSF-1 is a preferred way for performing the present invention. Other preferred embodiments include the administration of CSF-1 expressing plasmids, genetically modified CSF-1 molecules (e.g. showing enhanced activity or prolonged half life in the body (heart)) or the administration of cells with enhanced CSF-1 expression, e.g. by way of CSF-1 transfected cells (transgenic CSF-1) or by way of cells that have an enhanced (endogenous) CSF-1 production due to a changed regulation of the CSF-1 gene (e.g. by gene activation technology).

According to a preferred embodiment of the present invention, the pharmaceutical preparation further contains cells selected from myoblasts, fibroblasts, cardiomyocytes, stem cells, especially stem cells from bone marrow, from umbilical cord or circulating blood, endothelial cells or mixtures thereof. With such cells, a good substrate is provided whereupon CSF-1 can directly act for improving the heart tissue e.g. at the site of infarction.

The two points mentioned above can also be achieved simultaneously by providing a pharmaceutical preparation (further) that contains cells, preferably myoblasts, which have an increased expression of CSF-1, compared to normal cells myoblasts or cardiomyocytes. With such cells, additional administration of (exogenous) CSF-1 according to the present invention can be omitted; these cells can maintain suitable CSF-1 expression profiles for a sufficient time.

Accordingly, a pharmaceutical preparation containing CSF-1-transfected cells, especially CSF-1-transfected myoblasts, represents a specifically preferred embodiment of the present invention. Additional administration of CSF-1 may be performed in this case, but can also be omitted without losing effectiveness of the present invention.

The CSF-1 applied according to the present invention is preferably (CSF-1 expressing plasmids or constructs, recombinant CSF-1) human CSF-1, especially if human (but not exclusively, since human CSF-1 is effective in other species, especially mammals as well) patients are to be treated. Alternatively, also other forms of CSF-1 molecules may be used, e.g. from various animals, especially mammal sources, recombinant or non-recombinant, truncated or mutated forms of CSF-1 (as long as the CSF-1 activity characteristics are present (e.g., CSF-1 receptor, c-fms modification or macrophage recruitment).

Besides CSF-1, also other growth factors may be administered according to the present invention, preferably VEGF forms, e.g. VEGF-A, VEGF-B, VEGF-C, VEGF-D, bFGF, Angiopoietin-1, Angiopoietin-2, PDGF, PlGF, GM-CSF, or other cytokines or growth factors or mixtures thereof. However, according to a preferred embodiment, CSF-1 is the main or even exclusive pharmaceutically active substance applied to the specific heart region (however, also applied as e.g. CSF-1-transformed cell).

According to another aspect, the present invention relates to a pharmaceutical composition comprising cells selected from myoblasts, fibroblasts, cardiomyocytes, stem cells, especially stem cells from bone marrow, from umbilical cord or circulating blood, endothelial cells or mixtures thereof and CSF-1.

The present invention also provides a pharmaceutical composition comprising CSF-1-transfected cells, especially CSF-1-transfected myoblasts.

Another aspect of the present invention relates to a kit for treating heart failure as defined above, comprising
- CSF-1 and
- cells selected from myoblasts, fibroblasts, cardiomyocytes, stem cells, especially stem cells from bone marrow, from umbilical cord or circulating blood, endothelial cells or mixtures thereof.

This kit may also comprise
- CSF-1 and
- CSF-1 expressing plasmids or constructs and/or
- CSF-1-transfected cells, especially CSF-1-transfected myoblasts;
however, as mentioned above, also the cells alone (without additional CSF-1) are suitable.

According to another aspect, the present invention also relates to a method for treating or preventing heart failure in a patient comprising administering to said patient an effective amount of a pharmaceutical preparation comprising CSF-1.

Also the way of administration is - in principle - not critical but may in the individual case be dependent on the specific needs of the patient and the type of disorder. Preferred routes of application are: by direct, intramyocardial application of the pharmaceutical preparation into the heart by means of a catheter over the coronary artery, systemically, especially intravenously, by retarded administration (e.g. subcutaneous depots), or oral administration, or combinations of these application techniques.

Preferably, the effective amount of CSF-1 at the site of administration is upheld for at least 1 day, preferably at least 4 days, especially at least 7 days, preferentially up to 14 days or longer.

The invention is further described in the following examples and the figures, yet without being restricted thereto.

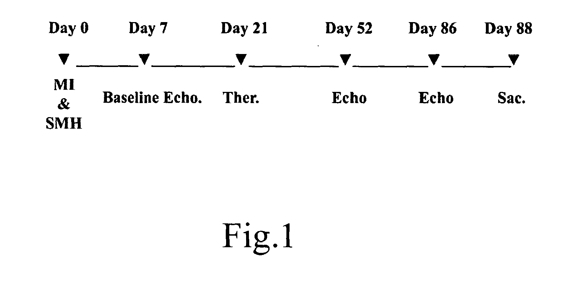

Figure 1 shows the protocol over the 3-month period. MI = myocardial infarction; SMH = skeletal muscle harvest; Ther. = therapy (intramyocardial injection of myoblasts or recombinant CSF-1 or CSF-1 expressing plasmids or culture medium (dulbecose modified Eagle medium: DMEM)); Echo = echocardiography; sac = sacrifice.

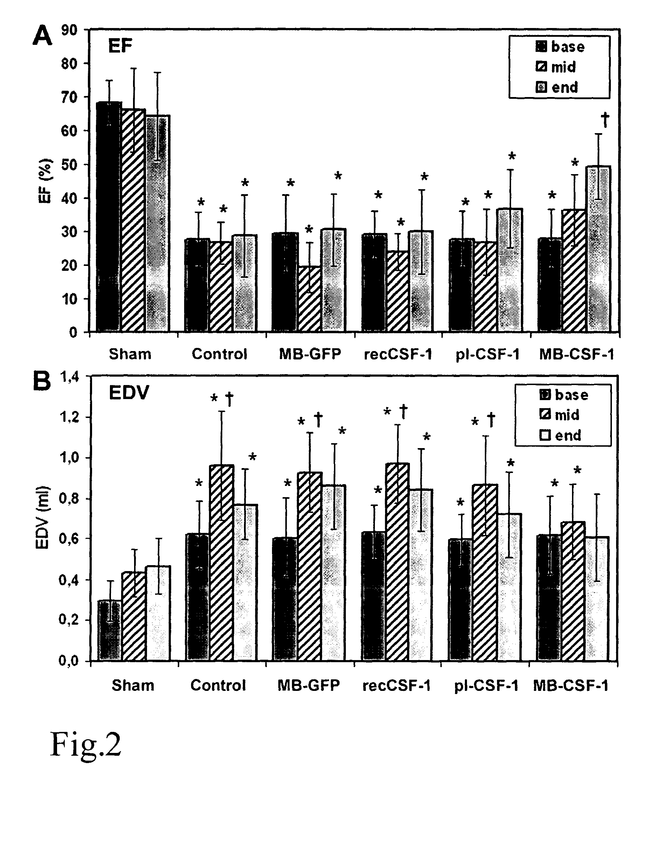

Fig. 2 shows functional cardiac assessment. Fig. 2A comparisons of left ventricular ejection fraction (EF) within each group at different time points. Base (dark gray bars) indicates baseline echocardiographic evaluation at day 7 after myocardial infarction (MI); mid (hatched bars) and end (light gray bars) indicate day 52 and day 86 echocardiographic assessment. Only CSF-1-transfected myoblasts (MB-CSF-1) improved cardiac EF significantly over time, while EF of green fluorescent protein (GFP)-transfected myoblasts (MB-GFP), the recombinant human CSF-1 (recCSF-1), CSF-1 expression plasmid (pl-CSF-1), and untreated control group remained inferior vs. sham operated rats (Sham).*, significantly different from Sham (P < 0.001), t significantly different from MB-CSF-1 on day 7 (P < 0.001) and day 52 (P = 0.007). Fig. 2B comparisons of left ventricular end-diastolic volume (EDV) within each group at different time points. Base (dark gray bars) indicates baseline echocardiographic evaluation at day 7 after myocardial infarction (MI); mid (hatched bars) and end (light gray bars) indicate day 52 and day 86 echocardiographic assessment. EDV was significantly improved in the MB-CSF-1 group on day 86 vs. sham operated animals. *, significantly different from the sham group on days 7, 52, and 86, respectively, in controls (P < 0.001; P < 0.001; P < 0.001), MB-GFP (P < 0.001; P < 0.001; P < 0.001), recCSF-1 (P < 0.001; P < 0.001; P < 0.001), pl-CSF-1 (P < 0.001; P < 0.001; P = 0.004), and in MB-CSF-1 (P < 0.001 and P = 0.004 vs. sham group on days 7 and 52, respectively). t significantly different from day 7 within group (*P* < 0.001, Control d52 vs. d7; *P* < 0.001 and *P* = 0.004, MB-GFP d52 and d86, respectively, vs. d7; P < 0.001, recCSF-1 d52 vs. d7; P < 0.001, pl-CSF-1 d52 vs. d7).

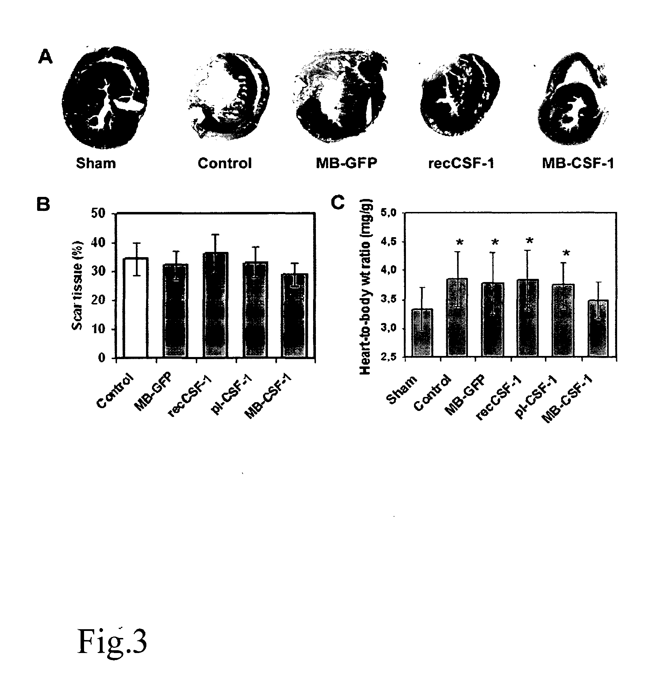

Fig. 3 shows that CSF-1-transfected myoblasts reduce scar tissue formation. Fig. 3A representative images of collagenous scar tissue in left ventricular myocardium by Goldner trichrome stain show a reduction in scar tissue in the MB-CSF-1 group compared with the other non-sham groups at 88 days post myocardial infarction. Fig. 3B quantitative morphometric analysis of Goldner trichrome stained myocardial scar tissue sections on day 88 after infarction in the control, MB-GFP, recCSF-1, pl-CSF-1, and MB-CSF-1 groups. Scar region was reduced in the MB-CSF-1 group. Fig. 3C heart-to-body weight ratio was significantly increased in all groups except for animals receiving MB-CSF-1 as compared to sham animals. *, significantly different from sham (P = 0.002, P = 0.012, P = 0.003, and P = 0.029 for controls, MB-GFP, recCSF-1, and pl-CSF-1, respectively).

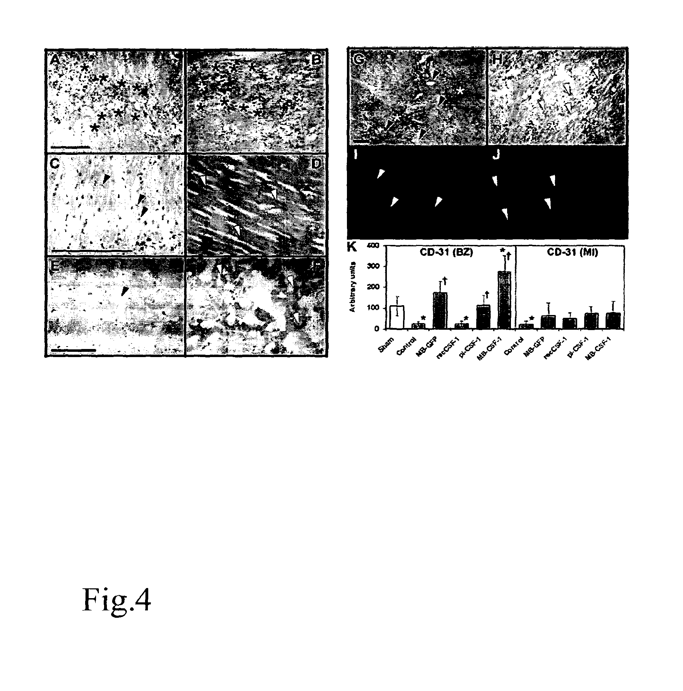

Fig. 4 shows integration and stimulation of angiogenesis by CSF-1-transfected myoblasts. Figs. 4A, B representative images of HE-stained myocardial sections, in both the MB-GFP (A) and MB-CSF-1 (B) groups, showing migration of transplanted myoblasts (white asterisks; migrating cells marked-off by black asterisks) of both groups into neighboring myocardial tissue, forming unstructured tissue within the myocardium. Figs. 4C-F HE-staining (C, D) and staining with skeletal muscle myosin heavy chain specific antibody (E, F) identifies integrated single myocytes (black and white arrowheads) in between the cardiac myofibers in MB-GFP (C, E) and MB-CSF-1 (D, F) treated animals. Figs. 4G-J representative images of HE-stained and endothelial cell specific marker CD-31-stained sections, in both the MB-GFP (G, I) and MB-CSF-1 (H, J) groups, reveal a large number of vessels (arrowheads) in areas with transplanted myoblasts (white asterisks). Bar = 100 µm. Fig. 4K quantification of protein expression levels of CD-31 Western blots in myocardial tissue of the border zone (BZ) and the myocardial infarction (MI) zone 88 days after myocardial infarction. CD-31 level is highest in the border zone of the MB-CSF-1 group followed by the MB-GFP group. In the MI zone, CD-31 expression levels of the MB-GFP, pl-CSF-1, and MB-CSF-1 groups were slightly higher vs. other groups. * significant different vs. sham (P = 0.020, P = 0.023, P < 0.001 for control, recCSF-1, and MB-CSF-1, respectively, in the BZ; P = 0.010 for control in MI); t significantly different vs. control (P < 0.001 for MB-GFP and MB-CSF-1, respectively, and P = 0.01 for pl-CSF-1 in the BZ).

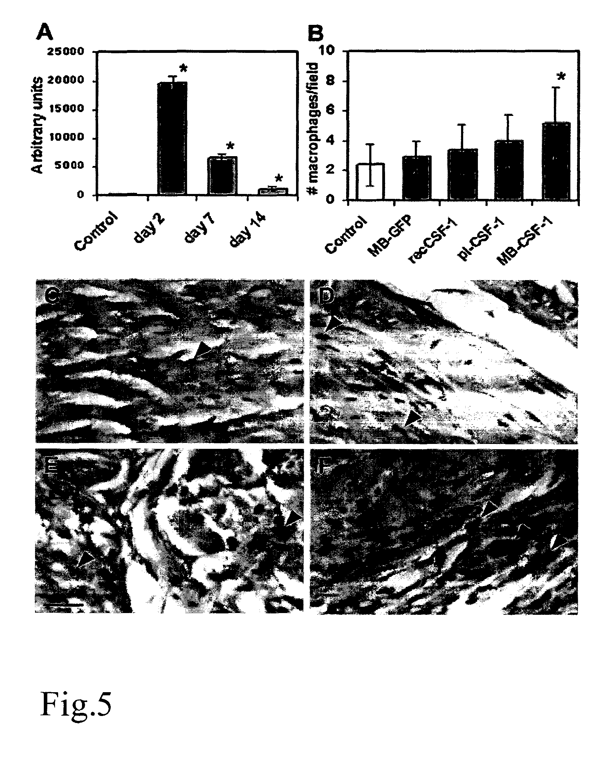

Fig. 5 shows that CSF-1 expressing myoblasts recruit macrophages. Fig. 5A CSF-1 mRNA expression levels over time following transfection of myoblasts with CSF-1 expression plasmid in vitro. CSF-1 mRNA levels are still elevated 14 days after transfection. *, significantly different from control (P < 0.0001). Fig. 5B quantitative histo-morphometric analysis, of myocardial tissue sections of the border zone on day 88 after infarction in the control, MB-GFP, recCSF-1, pl-CSF-1, and MB-CSF-1 groups stained with antibody to the macrophage marker protein F4/80. The density of F4/80 positive macrophages is increased following MB-CSF-1 treatment. *, significantly different from control (P = 0.032). Figs. 5C-F representative immunohistochemistry images of myocardial tissue sections of the border zone on day 88 after infarction in the control group (C), MB-GFP (D), recCSF-1 (E), and MB-CSF-1 (F) group stained with antibody to the macrophage marker protein F4/80. Arrowheads indicate macrophages stained positively with F4/80 antibody inside the myocardial tissue.
Calibration bar = 10 µm.

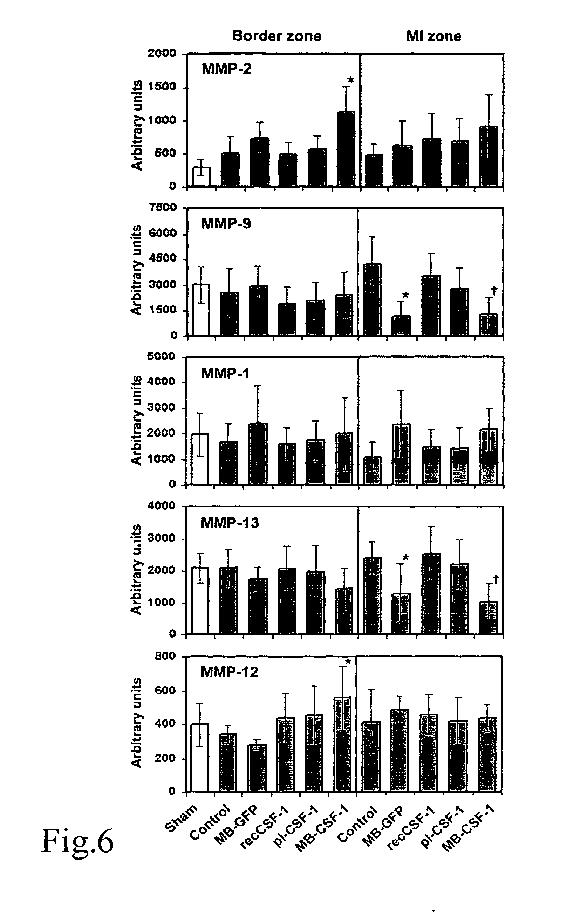

Fig. 6 shows differential expression of MMPs in myocardial tissue following treatment. Quantification of protein expression levels of MMP-2, MMP-9, MMP-1, MMP-13, and MMP-12 Western blots in myocardial tissue of the border zone and the myocardial infarction (MI) zone 88 days after myocardial infarction (67 days after start of therapy). MMP-2 expression is increased in the border zone of the MB-CSF-1 group vs. sham and control groups. Protein expression of MMP-9 of MB-GFP and MB-CSF-1 groups is reduced vs. control group in the MI zone. MMP-1 expression is unchanged, while expression of MMP-13 is reduced in the MI zone in the MB-GFP and MB-CSF-1 groups vs. control group. The MMP-12 level is upregulated in the border zone of the MB-CSF-1 group vs. control group. MMP-2: *, significantly different from sham (P < 0.0001) and control (P = 0.006); MMP-9: *, significantly different from control (P < 0.0001), and t, significantly different from control (P = 0.001); MMP-13: *, significantly different from control (P = 0.045), t, significantly different from control (*P* = 0.003); MMP-12: *, significantly different from control (*P* = 0.039).

### EXAMPLES

### Study concerning autologous transplantation of CSF-1-transfected myoblasts into myocardium attenuates ischemia-induced heart failure

This study was designed to examine whether myoblasts that are genetically engineered to overexpress CSF-1, then transferred to the failing heart following myocardial infarction, can improve cardiac function. The underlying rationale for these studies is two-fold. First, CSF-1 accelerates angiogenesis in vivo (18), affects macrophage-induced production of matrix metalloproteases (MMPs), and subsequently extracellular matrix (ECM) remodeling (19,20). Second, it has been shown that the proliferation of L6α1 rat myoblasts is regulated in an autocrine fashion by CSF-1 and that this regulation is lost as they differentiate to myotubes (21). Overexpression of CSF-1 may therefore enhance the proliferation of transplanted myoblasts. In the present study it is shown that transplantation of CSF-1-overexpressing myoblasts into the infarcted and failing rat heart attenuates myocardial remodeling, enhances angiogenesis, and improves significantly the cardiac function.

### Materials and Methods

**Animal groups, experimental design, operative procedure.** The experiments performed in this study were approved by the Institutional Animal Care and Use Committee at the University of Vienna. Male Sprague Dawley rats (Harlan, Borchen, Germany), weighing 280 g, were weighed and coded, and randomly assigned to experimental groups of n = 30. The intra- and post-operative mortality following myocardial infarction was 32% in average. The final number of animals with comparable baseline cardiac function in each group was n = 20. In groups 1-5, a myocardial infarction was induced and animals were treated with intramyocardial injection of human CSF-1-transfected myoblasts in group 1 (MB-CSF-1), GFP-transfected myoblasts in group 2 (MB-GFP), 10⁶ IU recombinant human CSF-1 in group 3 (recCSF-1), CSF-1 expression plasmid (pl-CSF-1) in group 4, and 150 µl culture medium in group 5 (control). Group 6 contained sham operated rats (sham).

Rats were anesthetized (ketamine hydrochloride/xylazine at 55/7.5 mg/kg, s.c.) and intubated tracheally with a 14-gauge i.v. catheter and ventilated with oxygen/isoflurane mixture at Pmax. inspir. of 20 mm Hg and Tinspir. at 0.6 sec. and Texpir. at 0.8 sec, before they underwent a left lateral thoracotomy in the 5th intercostal space. The left anterior descending branch of the coronary artery (LAD) was either ligated between the left atrium and the right pulmonary outflow tract with a 7-0 polypropylene snare (Ethicon, Somerville, NJ) (22) or left intact in sham procedure. Seven days after the operative procedure the left ventricular function was studied by two-dimensional echocardiography.

At day 21, an inferior lateral thoracotomy was performed; and adhesions were carefully released. The infarcted area was identified and 150 µl DMEM culture medium containing 5 x 10⁶ transfected myoblasts, or 150 µl culture medium alone, or 150 µl (22) Ringer's solution containing 10⁶ IU recombinant CSF-1, or 150 µl solution containing 10 µg pCIneo-CSF-1 expression plasmid (as described below) with Effecten transfection reagent (Qiagen, Hilden, Germany) at a ratio of 1 µg DNA to 6 µl Effecten, were injected into the infarcted myocardium using a 30 gauge needle with its tip curved to 45° to facilitate intramyocardial and to avoid transmyocardial injection. The injection volume was delivered to the central and bordering areas of infarcted myocardium by seven injections. Sham animals were re-thoracotomized only. On days 52 and 86, the left ventricular function was assessed again by echocardiography. Animals were sacrificed on day 88 and their hearts were obtained (Fig. 1).

**Isolation and culture of skeletal myoblasts.** Following the chest closure, the right anterior tibial muscle was removed and isolated from connective tissue. The muscle was rinsed and minced in phosphate buffered saline (PBS), and passed through a 100 µm meshed sieve (Cell Strainer Nylon; BD, Franklin Lakes, NJ, USA) to obtain a cell suspension free of debris. Cells were suspended in DMEM containing 10% fetal calf serum (FCS; PAA, Linz, Austria), 100 U/ml penicillin, 100 µg/ml streptomycin (Invitrogen, Carlsbad, CA, USA), and were plated initially in 75-cm² Falcon tissue culture flasks (Becton & Dickinson, Palo Alto, CA). Cells were maintained at 37° C in humidified air with 5% CO₂, and examined daily for confluency and viability. Adherent cells approaching confluency were harvested with 1x Trypsin-EDTA (Invitrogen) and subcultivated with a split ratio of 1:3.

**Skeletal muscle cell characterization.** Cultured cells were screened using myocyte specific anti desmin (Clone D33; DAKO, Glostrup, Denmark) and a fibroblast specific antibody (Clone 1B10; Harlan/Sera-Lab, Sussex, England). Cells were rinsed with tris-buffered-saline (TBS), fixed with acetone, and incubated with biotinylated primary antibodies (dilution 1:50). After rinsing with TBS, cells were incubated with streptavidin-peroxidase for 15 min, followed by addition of DAB for 5 min. Cells were analysed with a phase contrast microscope and SIS image analysis software (Münster, Germany). The proportion of myoblasts was calculated by dividing the number of desmin positive cells corrected to the total number of cells. The average percentage of myoblasts ranged from 70% to 81%.

**Plasmids, myoblast transfection and preparation for intramyocardial injection.** The GFP expression plasmid pEGFP-N1 was obtained from Clontech Laboratories (Palo Alto, CA). The expression vector has a cytomegalovirus (CMV) promoter that regulates GFP transcription in eukaryotic cells. Mouse CSF-1 cDNA was cloned from mouse L929 fibroblasts (23) by PCR and was ligated into pCIneo mammalian expression vector (Promega, Madison, WI) using EcoRI sites resulting in pCIneo-CSF-1. CSF-1 expression in this plasmid is driven by a CMV promoter. The construct was characterized by restriction digests and purified by DNA columns (Qiagen). Skeletal myoblasts were grown to 70% confluency before they were transfected with the pCIneo-CSF-1 construct or with the GFP expression vector pEGFP-N1 using the non-liposomal lipid transfection reagent Effecten (Qiagen) at a ratio of 1 µg DNA to 30 µl Effecten. CSF-1 expression was tested by real-time RT-PCR and GFP expression was examined by fluorescence microscopy. Myoblasts were harvested 24 hours after transfection by trypsinization, rinsed with PBS, counted, and their viability was assessed with 0.4% trypan blue (Gibco BRL, Gaithersburg, MD). Cells were rinsed and kept in DMEM on ice until transplantation.

**Quantitative real-time RT-PCR.** Samples were homogenized in liquid nitrogen and processed for PCR as described earlier (24). The primer sequences (sense/antisense) were: 
mouse CSF-1:   5´-CATCTCCATTCCCTAAATCAAC-3´/
                5´-ACTTGCTGATCCTCCTTCC-3´;
 
rat GAPDH:     5´-ACCCCTTCATTGACCTCAAC-3´/
               5´- ACGACATACTCAGCACCAGC-3´.
 LCDA Version 3.1.102 was used for PCR data analysis (Roche, Mannheim, Germany). Measurements were performed three times.

**Myocardial functional assessment.** Two-dimensional and M-mode measurements were performed in anesthetized rats (ketamine/xylazine as described above). A 15 MHz (15L8) linear array scan head equipped with SonoCT® -technology (ATL, HDI 5000; Philips, Best, Netherlands; allowing a 160-Hz maximal frame rate) was used. In supine or left lateral position, parasternal long-axis views were obtained, the mitral and aortic valves and the apex of the heart were visualized, and recorded. Measurements of maximal left ventricular (LV) long-axis lengths (L) and endocardial area tracing were performed from digital images. LV end-diastolic (LVEDV) and LV end-systolic volume (LVESV) were calculated by the single-plain area-length method (25): V = 8 x a²/(3 x n x L). Left ventricular ejection fraction (LVEF) was then calculated by using the formula LVEF = (LVEDV - LVESV)/ LVEDV. Echocardiographic measurements were performed by two blinded investigators from at least three consecutive cardiac cycles to minimize beat to beat variability.

**Histology, immunocytochemistry, heart-to-body weight ratio.** At the end of the study animals were weighed and their hearts were removed. After rapid rinse in PBS, the hearts were weighed and the heart-to-body weight ratio (mg/g) was estimated (26). The ventricles were isolated and cross-sectioned at the midpoint of their long axis, before they were either frozen in tissue freezing medium or immersion-fixed in 5% NB formalin. Paraffin-embedded specimens were used for H&E, Ki-67 and CD-31 (20) (endothelial cell proliferation assay; Dako), macrophage immunostaining (anti-F4/80 rat monoclonal antibody; Caltag Laboratories, Burlingame, CA), as well as Goldner trichrome collagen staining. Deparaffinized sections were preincubated with 3 % H₂O₂ and horse serum to block endogenous peroxidase and antigen activity, prior to incubation with the Ki-67 and CD-31 primary antibody or F4/80 antibody (27) (1:50 dilution). Biotinylated secondary antibody (Vector Laboratories, Burlingame, CA) was used to detect positive staining followed by streptavidin-biotin peroxidase complex. Frozen sections were used to detect skeletal muscle myosin heavy chain (My-32; Biogenex, San Ramon, CA, USA) and vascular endothelial-specific CD-31 (TCS, Buckingham, UK). The first antibody was detected using a Vecastain ABC ELITE peroxidase kit (Vector); and the reaction was developed with DAB (Sigma). Negative controls were made by omitting the first antibody. Digitalized images were generated and morphometry was carried out (20).

**Western Blotting.** Tissue samples were lyzed in solubilization buffer and Complete™ - EDTA-free Protease Inhibitor Cocktail (Roche, Mannheim, Germany). Tissue lysates (50 µg/lane) were separated by 7.5% SDS-PAGE prior to electrophoretic transfer onto Hybond C super (Amersham Pharmacia Biotech, Buckinghamshire, UK). The blots were probed with antibodies against CD31 (mouse monoclonal, US Biological, Swampscott, MA), MMP-1 (mouse monoclonal, Oncogene, Cambridge, MA), MMP-2 (goat polyclonal, Santa Cruz Biotechnology, Santa Cruz, CA), MMP-9 (rabbit polyclonal, Chemicon, Temecula, CA), MMP-12 (goat polyclonal, Santa Cruz Biotechnology), MMP-13 (rabbit polyclonal, Neomarkers, Fremont, CA), TIMP-1 (rabbit polyclonal, Chemicon), TIMP-2 (rabbit polyclonal, Chemicon), TIMP-3 (rabbit polyclonal, Chemicon), and TIMP-4 (rabbit polyclonal, Chemicon) prior to incubation with horseradish peroxidase-conjugated secondary antibodies (Amersham) (20). Proteins were immunodetected on the membrane using chemiluminescence (Supersignal-West-Femto, Pierce, Rockford, IL) and specific protein bands were quantified using Easy plus Win 32 software (Herolab, Wiesloch, Germany).

**Statistical analyses.** Analysis of variance with Bonferroni t-test was used to compare the data between the groups. Data are expressed as means ± SD. P values of < 0.05 were considered to indicate statistical significance.

### Results

### CSF-1-transfected myoblast improved myocardial function.

Baseline LVEF on day 7 (d7) was not different between controls (27,6%), MB-GFP (29,5%), recCSF-1 (29%), pl-CSF-1 (27,7%), MB-CSF-1 (28%), although it was significantly lower vs. the sham group (68%; P < 0.001). LVEF in all treated animals on d52 did not differ significantly from LVEF on d7, although the average LVEF in the MB-CSF-1 group on d52 was the only one increased. LVEF on d86 was significantly lower in all treatment groups (P < 0.001). However, LVEF on d86 for MB-CSF-1 group (49%) was significantly increased vs. d7 (P < 0.001) and vs. d52 (P = 0.007; Fig. 2A). Only the pl-CSF-1 group showed an increased LVEF on d86 similar to the changes within the MB-CSF-1 group, although not reaching statistical significance vs. days 7 and 52 (Fig. 2A).

The LVEDV was significantly elevated in controls (0.63 ± 0.17, *P* < 0.001; 0.96 ± 0.27, *P* < 0.001; 0.77 ± 0.18, *P* < 0.001), MB-GFP (0.61 ± 0.19, *P* < 0.001; 0.93 ± 0.20, *P* < 0.001; 0.86 ± 0.21, *P* < 0.001), recCSF-1 (0.64 ± 0.14, *P* < 0.001; 0.97 ± 0.19, *P* < 0.001; 0.84 ± 0.20, *P* < 0.001), and pl-CSF-1 (0.60 ± 0.13, *P* < 0.001; 0.87 ± 0.25, *P* < 0.001; 0.72 ± 0.21, *P* = 0.004) vs. the sham group (0.30 ± 0.10; 0.43 ± 0.11, 0.47 ± 0.14), on days 7, 52, and 86, respectively. In contrast, LVEDV was significantly improved in the MB-CSF-1 group on d86 (0.61 ± 0.22, P = 1.00 vs. sham d86), although LVEDV on days 7 and 52 was still significantly higher in MB-CSF-1 (0.62 ± 0.19, P < 0.001; 0.69 ± 0.19, P = 0.004;) vs. sham animals (Fig. 2B). These data indicated that CSF-1-transfected myoblasts significantly improved cardiac ejection fraction and attenuated myocardial infarction-induced ventricular dilation.

### CSF-1-transfected myoblasts attenuate myocardial remodeling.

Despite similar initial reductions in LVEF by 1 week in the non-sham groups, the mean proportion of scar tissue/normal left ventricular myocardium (as defined by Goldner trichrome stain) (Fig. 3A) at 88 days post myocardial infarction was 34%, 32%, 36%, 33% in control, MB-GFP, rec-CSF-1, and pl-CSF-1 group, respectively, and was reduced to 29% in rats receiving MB-CSF-1 (Fig. 3B). In concert with these histologic findings, heart-to-body weight ratio was significantly increased in controls (3.86 ± 0.45 mg/g; *P* = 0.002), in MB-GFP group (3.78 ± 0.49 mg/g; *P* = 0.012), in recCSF-1 group (3.84 ± 0.47 mg/g; *P* = 0.003), and pl-CSF-1 group (3.74± 0.38; *P* = 0.029) but not in rats receiving MB-CSF-1 (3.48 ± 0.29 mg/g) as compared to sham animals (3.33± 0.36 mg/g) (Fig. 3C). Together, these results further indicate that treatment with CSF-1 overexpressing myoblasts attenuated myocardial remodeling and reduced ventricular dilation by 12 weeks.

**CSF-1-transfected myoblasts migrate, align, and stimulate angiogenesis.** At 88 days post-myocardial infarction, transferred myoblasts of both MB-GFP and MB-CSF-1 groups had migrated into neighboring myocardial tissue, but myoblasts had not formed an organized tissue within the myocardium (Fig. 4A,B). However, single myocytes were found in between the cardiac myofibers in MB-GFP and MB-CSF-1 treated animals, which were structurally integrated and aligned into the recipient myocardial tissue. No fibrotic reaction was seen surrounding these transplanted cells (Figs. 4C-F). In addition, in both the MB-GFP and MB-CSF-1 group a large number of vessels could be found in areas with transplanted myoblasts (Figs. 4G-J). Consistent with these observations, Western blot analysis of the endothelial cell specific marker CD-31 revealed a significant higher expression levels in the border zone of the MB-CSF-1 group only (P < 0.001 vs. sham), while CD-31 levels of MB-GFP and pl-CSF-1 groups were unchanged, and that of control (P = 0,020 vs. sham) and recCSF-1 (P = 0,023 vs. sham) groups even significantly reduced vs. sham animals. In the MI zone, the average CD-31 expression levels of the MB-GFP, pl-CSF-1, and MB-CSF-1 groups were higher than in control and rec-CSF-1 groups, but were not significantly different (Fig. 4K). These findings indicate that myoblasts have the ability to migrate and integrate into the myocardial tissue, and that CSF-1 expressing myoblasts are more efficient than GFP-transfected myoblasts in stimulating angiogenesis.

**Efficient myoblast transfection with GFP and CSF-1 plasmids increases macrophage recruitment.** Isolated myoblasts were cultured and non-viral transfection efficiency was optimized by using a GFP expression plasmid. Optimal conditions obtained from these experiments were further used for final transfection of myoblasts with GFP and CSF-1, designated for intramyocardial transplantation. Transfection efficiency was about 70% 2 days after transfection. The percentage of myoblasts expressing GFP declined to 45% and 20% on days 7 and 14, respectively (data not shown). To examine the CSF-1 gene expression over time, real-time RT-PCR analysis in transfected myoblasts was performed.

CSF-1 was overexpressed significantly on days 2, 7, and 14 following transfection as compared to untransfected control myoblasts, respectively (P < 0.001, Fig. 5A). After treatment with CSF-1 transfected myoblasts, a significant increase in the number of macrophages was observed in the border zone 88 days post myocardial infarction compared to control group (P = 0.032). In contrast, neither treatment with recCSF-1, pl-CSF-1 nor with MB-GFP resulted in significant changes in the number of macrophages as determined by macrophage specific staining with F4/80 antibody, although macrophage were increased after pl-CSF-1 treatment (Figs. 5B-5F). These experiments indicate that the transfected myoblasts sustain the ability to overexpress CSF-1 for at least 2 weeks and that CSF-1 expressing myoblasts are able to attract macrophages.

**CSF-1 affects the myocardial MMP expression**. MMP-2 protein levels determined by Western blotting at 88 days post myocardial infarction (67 days post treatment), respectively, were significantly increased in the border zone of the MB-CSF-1 group compared to sham group (P < 0.0001) and controls (p = 0.006) (Fig. 6). No significant changes compared with controls were found for the MB-GFP, recCSF-1, and pl-CSF-1 groups in the border zone. In the MI zone, no significant changes were observed between groups. In contrast, MMP-9 protein expression remained unchanged in the border zone, but was significantly reduced in the MI zone for the MB-CSF-1 (P = 0.001) and MB-GFP (P < 0.0001) groups vs. control group. MMP-1 protein expression was not significantly altered for these groups in both border and MI zones, while MMP-13 levels, similar to MMP-9, were found to be significantly reduced within the MI zone in the MB-CSF-1 (P = 0.003) as well as the MB-GFP (P < 0.045) group vs. the control group. Macrophage specific MMP-12 protein expression was significantly increased in the border zone of the MB-CSF-1 group vs. the control (P = 0.039) group, but was not altered in the MI zone. In contrast to these findings with MMP expression, protein expression of TIMPs (TIMP-1, -2, -3, and-4) did not significantly change in the border or MI zone of any group (data not shown). These data indicate that a specific expression pattern of MMPs is displayed in the myocardial border zone of CSF-1 transfected myoblasts.

### Discussion

Studies in animal models of myocardial infarction and in patients with ischemic heart failure have demonstrated the feasibility of myoblast transplants for myocardial failure and the ability of the transplanted myoblasts to survive in the heart (9-12,16). This study was designed to enhance the survival and the alignment of grafted myoblasts and to improve the angiogenesis following myoblast transfer to the failing heart. It is evident that myosin heavy chain is not expressed in myoblasts early after engraftment; however, with time, cells fuse into myotubes, differentiate into myofibers, and express myosin heavy chain (12). Therefore, those areas that stained positive for myosin heavy chain within the grafts represented myoblasts that have survived transplantation and gone on to fuse and form myofibers. This process took place in both GFP-MB and CSF-1-MB group; however, the percentage of myoblasts that survived was significantly higher in CSF-1-MB group. Myoblast transfer 7 days after myocardial infarction has reportedly the ability to improve cardiac function (22), however, the same procedure failed in the present setting of ischemic heart failure. In contrast myoblasts overexpressing CSF-1 did improve the cardiac function in the present protocol, indicating the benefit of CSF-1 for cardiomyoplasty in the setting of ischemic heart failure.
Previous studies utilizing the rat L6α1 myoblast system have demonstrated that CSF-1 regulates myoblast proliferation in an autocrine fashion, but that expression of both CSF-1 and the CSF-1R is lost upon differentiation of the myoblast to myotubes. Indeed, the induced expression of CSF-1R antisense RNA in these cells blocked myoblast proliferation and accelerated myogenic differentiation (21). Thus one reason for the increased survival of myoblasts in the CSF-1-MB group is that increased myoblast expression of CSF-1 can be expected to increase their proliferative capacity, giving them a significant growth advantage in regions of myocardial infarction. Interestingly, an effect of exogenous CSF-1 (the recCSF-1 group) on myoblast proliferation was not expected since evidence indicates the autocrine regulation of myoblast proliferation by CSF-1 is apparently intracellular (21). Another reason for the effectiveness of the CSF-1-MB group is that local production of CSF-1 has been shown to recruit and sustain many tissue macrophage populations which possess trophic and scavenger functions that are important for normal tissue development (28,29). These functions of the macrophages are also quite relevant to the changes observed during recovery from myocardial infarction (see below).

It is noteworthy that the majority of myofibers surviving in the heart have aligned in parallel with the host myocardial fibers in the CSF-1-MB group, and that the percentage of such myofibers was significantly higher than in the GFP-MB group. This observation suggests a potential for productive synchronous contraction, or structural enhancement, to have contributed to the observed remodeling and improvements in cardiac function. In line with this, electron microscopy (30) demonstrated the capability of CSF-1-MBs to form proper gap junctions. Additional indirect evidence for the long term survival and alignment of myoblasts in the failing heart is that although the size of the scar tissue was moderately reduced, the ejection fraction was improved and the left ventricular dilation ameliorated significantly in CSF-1-MB group. The myocardial matrix metalloprotease (MMP) profile in the CSF-1-MB group also favors the hypothesis that CSF-1-induced recruitment of the macrophages has contributed to the suppression of the cardiac tissue remodeling, most likely by improvement of the myoblast integration in and around the infarcted myocardial zone. On the other hand, the potential for myoblast transplants to regenerate myocardial tissue following infarction depends on migration, nourishment, and spatially appropriate growth of myoblasts. Requisite to these objectives is a neovasculature, proper MMP activity, and fibrillar collagen scaffolding. In the border zone, the number of vessels and expression of endothelial cell-specific CD31 was increased in both myoblast transplant groups. However, the vascular network formed at the infarct site was highest for the MB-CSF-1 group, indicating an additional beneficial angiogenic effect of myoblast-derived CSF-1, thereby facilitating nourishment of cells for their growth and setting the stage for restoring function of the infarcted myocardium. Consistent with these angiogenic effects, a recent publication suggests that CSF-1 stimulates monocytes, progenitors of macrophages, to secrete biologically active VEGF (31).

Scar and collagen areas were smaller in the MB-CSF-1 group than in the other groups. In line with this, MMP-2 (gelatinase-A) and macrophage specific MMP-12 (metalloelastase) were overexpressed in the border zone of MB-CSF-1 treated animals. Besides overexpression of these MMPs, the expression of both MMP-9 (gelatinase-B) and MMP-13 (interstitial collagenase) expression was reduced in the infarction zone in both the MB-GFP and the MB-CSF-1 group 12 weeks post myocardial infarction. Previous studies reported that MMPs were increased in postinfarction heart failure models, leading to LV dilation and that MMP-inhibitors beneficially affected cardiac remodeling and function (32,33). However, the excessive fibrosis without contractility or relaxation could accelerate cardiac remodeling and decrease cardiac function, as seen in ischemic or idiopathic dilated cardiomyopathy. In such cases, an increase in the MMP family and proteolysis of excess collagen may be a protective mechanism. In fact, this concept is supported by findings that an inhibition of MMPs caused cardiac failure (34), and that targeted deletion of MMP 9 attenuated LV remodeling and collagen accumulation via overexpression of MMP-2 and MMP-13 (35). Furthermore, the importance of appropriate expression of MMPs has been demonstrated by experiments in mice showing that acute myocardial infarction resulted in cardiac rupture in mice lacking MMP-3 (stromelysin-1) or MMP-12, while lack of MMP-9 partially protected against rupture (34).

Relevant to the importance of differential expression of MMPs, specific changes in MMP-levels corresponding to the remote, transition, and infarction regions occurring after myocardial infarction have been described recently (36). Consistent with these observations, the present data indicate that alteration of region- and type-specific distribution of MMPs within the myocardium after infarction by CSF-1 producing myoblasts is associated with improved cardiac function. CSF-1 is likely to exert its effects on MMP-expression via enhanced recruitment of macrophages and regulation of their MMP production. This assumption is supported by previous findings in tumors in which CSF-1 was shown to enhance MMP expression and macrophage recruitment (37). Moreover, macrophage recruitment may be related to increased absorption of necrotic tissues by phagocytosis. The failure of the recombinant CSF-1 group to beneficially affect remodeling and cardiac function compared with the MB-CSF-1 group may be due to prolonged expression of CSF-1 by myoblasts, resulting in the maintenance of increased local CSF-1 concentrations for several weeks. The fact that transfection of the myocardium with CSF-1 plasmids slightly improved cardiac function, resulting in CSF-1 over-expression for at least 10 days, further supports this hypothesis.

In conclusion, there are multiple reasons for the effectiveness of repair by transplanted, autologous, CSF-1-transfected myoblasts in the present model of ischemic heart failure in rats. These include improved long term survival, alignment, and proliferative capacity of myoblasts, increased angiogenesis and secondary to increased macrophage migration toward the infarcted myocardium, specific changes of the MMP expression pattern.

References:
1. Mahon, N.G. et al. Hospital mortality of acute myocardial infarction in the thrombolytic era. Heart 81, 478-482 (1999).
2. Colucci, W.S. Molecular and cellular mechanisms of myocardial failure. Am. J. Cardiol. 80, 15L-25L (1997).
3. Itescu S, Schuster MD, Kocher AA. New directions in strategies using cell therapy for heart disease. J Mol Med. 2003;81:288-96.
4. Orlic D, Kajstura J, Chimenti S, Jakoniuk I, Anderson SM, Li B, Pickel J, McKay R, Nadal-Ginard B, Bodine DM, Leri A, Anversa P. Bone marrow cells regenerate infarcted myocardium. Nature. 2001 Apr 5;410(6829):701-5.
5. Kocher AA, Schuster MD, Szabolcs MJ, Takuma S, Burkhoff D, Wang J, Homma S, Edwards NM, Itescu S. Neovascularization of ischemic myocardium by human bone-marrow-derived angioblasts prevents cardiomyocyte apoptosis, reduces remodeling and improves cardiac function. Nat Med. 2001 Apr;7(4):430-6.
6. Menasche P, Hagege AA, Vilquin JT, Desnos M, Abergel E, Pouzet B, Bel A, Sarateanu S, Scorsin M, Schwartz K, Bruneval P, Benbunan M, Marolleau JP, Duboc D. Autologous skeletal myoblast transplantation for severe postinfarction left ventricular dysfunction. J Am Coll Cardiol. 2003; 41:1078-83.
7. R.C.J. Chiu, A. Zibaitis and R.L. Kao, Cellular cardiomyoplasty: myocardial regeneration with satellite cell implantation. Ann Thorac Surg 60 (1995), 12-18.
8. C.E. Murry, R.W. Wiseman, S.M. Schwartz and S.D. Hauschka, Skeletal myoblast transplantation for repair of myocardial necrosis. J Clin Invest 98 (1996), 2512-2523.
9. D.A. Taylor, B.Z. Atkins, P. Hungspreugs et al., Regenerating functional myocardium: improved performance after skeletal myoblast transplantation. Nat Med 4 (1998), 929-933.
10. M. Scorsin, A.A. Hagège, J.-T. Vilquin et al., Comparison of the effects of fetal cardiomyocytes and skeletal myoblast transplantation on postinfarction left ventricular function. J Thorac Cardiovasc Surg 119 (2000), 1169-1175.
11. B. Pouzet, S. Ghostine, J.-T. Vilquin et al., Is skeletal myoblast transplantation clinically relevant in the era of angiotensin-converting enzyme inhibitors?. Circulation 104 Suppl I (2000), I223-I228.
12. M. Jain, H. DerSimonian, D.A. Brenner et al., Cell therapy attenuates deleterious ventricular remodeling and improves cardiac performance after myocardial infarction. Circulation 103 (2001), 1920-1927.
13. P. Menasché, A.A. Hagège, M. Scorsin et al., Myoblast transplantation for heart failure. Lancet 357 (2001), 279-280.
14. El Oakley RM, Ooi OC, Bongso A, Yacoub MH.Myocyte transplantation for myocardial repair: a few good cells can mend a broken heart. Ann Thorac Surg. 2001;71:1724-33.
15. Hassink RJ, Brutel de la Riviere A, Mummery CL, Doevendans PA. Transplantation of cells for cardiac repair. J Am Coll Cardiol. 2003;41:711-7.
16. Pagani FD, DerSimonian H, Zawadzka A, Wetzel K, Edge AS, Jacoby DB, Dinsmore JH, Wright S, Aretz TH, Eisen HJ, Aaronson KD. Autologous skeletal myoblasts transplanted to ischemia-damaged myocardium in humans. Histological analysis of cell survival and differentiation. : J Am Coll Cardiol. 2003;41:879-88.
17. Ghostine S, Carrion C, Souza LC, Richard P, Bruneval P, Vilquin JT, Pouzet B, Schwartz K, Menasche P, Hagege AA. Longterm efficacy of myoblast transplantation on regional structure and function after myocardial infarction. Circulation. 2002;106:I131-6.
18.Aharinejad, S., Marks, S. C. Jr., Bock, P., Mason-Savas, A., MacKay, C. A., Larson, E. K., Jackson, M. E., Luftensteiner, M., and Wiesbauer, E. CSF-1 treatment promotes angiogenesis in the metaphysis of osteopetrotic (toothless, *t1*) rats. Bone, 16:315-324, 1995.
19. Tojo N, Asakura E, Koyama M, Tanabe T, Nakamura N.Effects of macrophage colony-stimulating factor (M-CSF) on protease production from monocyte, macrophage and foam cell in vitro: a possible mechanism for anti-atherosclerotic effect of M-CSF. Biochim Biophys Acta. 1999 Dec 9;1452(3):275-84.
20. Aharinejad S, Abraham D, Paulus P, Abri H, Hofmann M, Grossschmidt K, Schafer R, Stanley ER, Hofbauer R. Colony-stimulating factor-1 antisense treatment suppresses growth of human tumor xenografts in mice. Cancer Res. 2002; 62:5317-5324.
21. Borycki, A-G., Smadja, F., Stanley, E.R. and Leibovitch, S.A. Colony-Stimulating Factor 1 (CSF-1) is involved in an autocrine growth control of rat myogenic cells. Exp. Cell Res. 1995, 218: 213-222.
22. Pouzet B, Vilquin JT, Hagege AA, Scorsin M, Messas E, Fiszman M, Schwartz K, Menasche P. Intramyocardial transplantation of autologous myoblasts: can tissue processing be optimized? Circulation. 2000;102(19 Suppl 3):III210-5.
23. Ladner MB, Martin GA, Noble JA, Wittman VP, Warren MK, Mc-Grogan M, Stanley ER. cDNA cloning and expression of murine macrophage colony-stimulating factor from L929 cells. Proc Natl Acad Sci U S A. 1988;85:6706-10.
24. Abraham D, Hofbauer R, Schaefer R, et al. Selective downregulation of VEGF-A(165), VEGF-R(1), and decreased capillary density in patients with dilative but not ischemic cardiomyopathy. Circ Res 2000;87:644-7.
25. Schiller NB, Shah PM, Crawford M, DeMaria A, Devereux R, Feigenbaum H, Gutgesell H, Reichek N, Sahn D, Schnittger I, et al. Recommendations for quantitation of the left ventricle by two-dimensional echocardiography. American Society of Echocardiography Committee on Standards, Subcommittee on Quantitation of Two-Dimensional Echocardiograms. J Am Soc Echocardiogr. 1989;2:358-67.
26. Gomez AM, Schwaller B, Porzig H, Vassort G, Niggli E, Egger M. Increased exchange current but normal Ca2+ transport via Na+-Ca2+ exchange during cardiac hypertrophy after myocardial infarction. Circ Res. 2002 ;91:323-30.
27. Lin, E. Y., Nguyen, A. V., Russel, R. G., and Pollard, J. W. Colony-stimulating factor-1 promotes progression of mammary tumors to malignancy. J. Exp. Med., *193:* 727-739, 2001.
28. Cecchini, M.G., Dominguez, M.G., Mocci, S., Wetterwald, A., Felix, R., Fleisch, H., Chisholm, O., Pollard, J.W., Hofstetter, W. and Stanley, E.R. (1994) Role of colony stimulating factor-1 in the establishment and regulation of tissue macrophages during post-natal development of the mouse. Development 120: 1357-1372.
29. Dai X-M, Zong XH, Sylvestre V and Stanley ER. (2004) Incomplete restoration of colony stimulating factor-1 (CSF-1) function in CSF-1-deficient *Csf1*^{*op*}/*Csf1*^{*op*} mice by transgenic expression of cell surface CSF-1. Blood 103: 1114-1123.
30. H. Reinecke, G.H. MacDonald, S.D. Hauschka and C.E. Murry, Electromechanical coupling between skeletal and cardiac muscle. Implications for infarct repair. *J Cell Biol* 149 (2000), pp. 731-740.
31. Eubank TD, Galloway M, Montague CM, Waldman WJ, Marsh CB. M-CSF induces vascular endothelial growth factor production and angiogenic activity from human monocytes. *J Immunol* 2003;171:2637-43.
32. Spinale FG. Matrix metalloproteinases: regulation and dysregulation on the failing heart. *Circ Res.* 2002; 90: 520-530.
33. Lindsey ML, Mann DL, Entman ML, Spinale FG. Extracellular matrix remodeling following myocardial injury. Ann Med. 2003;35 (5):316-26.
34. Heymans S, Luttun A, Nuyens D, Theilmeier G, Creemers E, Moons L, Dyspersin GD, Cleutjens JP, Shipley M, Angellilo A, Levi M, Nube O, Baker A, Keshet E, Lupu F, Herbert JM, Smits JF, Shapiro SD, Baes M, Borgers M, Collen D, Daemen MJ, Carmeliet P. Inhibition of plasminogen activators or matrix metalloproteinases prevents cardiac rupture but impairs therapeutic angiogenesis and causes cardiac failure. Nat Med. 1999;5:1135-42.
35. Ducharme A, Frantz S, Aikawa M, et al. Targeted deletion of matrix metalloproteinase-9 attenuates left ventricular enlargement and collagen accumulation after experimental myocardial infarction. *J Clin Invest.* 2000; 106: 55-62
36. Wilson EM, Moainie SL, Baskin JM, Lowry AS, Deschamps AM, Mukherjee R, Guy TS, St John-Sutton MG, Gorman JH 3rd, Edmunds LH Jr, Gorman RC, Spinale FG. Region- and type-specific induction of matrix metalloproteinases in post-myocardial infarction remodeling. Circulation. 2003 Jun 10;107(22):2857-63
37. Aharinejad S, Paulus P, Sioud M, Hofmann M, Zins K, Schafer R, Stanley ER, Abraham D. Colony-stimulating factor-1 blockade by antisense oligonucleotides and small interfering RNAs suppresses growth of human mammary tumor xenografts in mice. Cancer Res. 2004 Aug 1;64(15):5378-84.

## Claims

1. Use of CSF-1 for the preparation of a pharmaceutical preparation for the treatment of ischemic heart diseases and heart failure.

2. Use according to claim 1, wherein the heart failure is selected from coronary heart disease associated with myocardial ischemia or non-ischemic forms of cardiomyopathy, such as dilative, hypertrophic, or restrictive cardiomyopathy.

3. Use according to any one of claims 1 to 2, wherein the pharmaceutical preparation contains a depot form of CSF-1.

4. Use according to any one of claims 1 to 3, wherein the pharmaceutical preparation further contains cells selected from myoblasts, fibroblasts, cardiomyocytes, stem cells, especially stem cells from bone marrow, from umbilical cord or circulating blood, endothelial cells or mixtures thereof.

5. Use according to any one of claims 1 to 4, wherein the pharmaceutical preparation contains cells, preferably myoblasts, which have an increased expression of CSF-1, compared to normal cells (myoblasts).

6. Use according to any one of claims 1 to 5, wherein the pharmaceutical preparation contains CSF-1-transfected cells, especially CSF-1-transfected myoblasts.

7. Use according to any one of claims 1 to 6, wherein the CSF-1 is recombinant human CSF-1, or a CSF-1 expressing plasmid or construct (viral or non-viral, e.g. transposon-based vectors).

8. Use according to any one of claims 1 to 7, wherein the pharmaceutical preparation further contains additional growth factors, preferably VEGF forms, e.g. VEGF-A, VEGF-B, VEGF-C, VEGF-D, bFGF, Angiopoietin-1, Angiopoietin-2, PDGF, PlGF, GM-CSF, or other cytokines or growth factors or mixtures thereof.

9. Pharmaceutical composition comprising cells selected from myoblasts, fibroblasts, cardiomyocytes, stem cells, especially stem cells from bone marrow, from umbilical cord or circulating blood, endothelial cells or mixtures thereof and CSF-1.

10. Pharmaceutical composition comprising CSF-1-transfected cells, especially CSF-1-transfected myoblasts.

11. Kit for treating ischemic heart disease or heart failure comprising
- CSF-1 and
- cells selected from myoblasts, fibroblasts, cardiomyocytes, stem cells, especially stem cells from bone marrow, from umbilical cord or circulating blood, endothelial cells or mixtures thereof.

12. Kit for treating ischemic heart disease or heart failure as defined in any one of claims 1 to 2, comprising
- CSF-1 and
- CSF-1-transfected cells, especially CSF-1-transfected myoblasts.

13. Method for treating ischemic heart disease or heart failure as defined in claims 1 to 2 in a patient comprising administering to said patient an effective amount of a pharmaceutical preparation comprising CSF-1.

14. Method according to claim 13, wherein the heart failure is a failure as defined in claim 2.

15. Method according to claim 13 or 14, wherein the pharmaceutical preparation is a preparation as defined in any one of claims 3 to 8.

16. Method according to any one of claims 13 to 15, wherein said administration is performed by direct intramyocardial application of the pharmaceutical preparation, by means of a catheter over the coronary artery, systemically, especially intravenously, by retarded administration (e.g. subcutaneous depots), or oral administration or combinations of these application techniques.

17. Method according to any one of claims 13 to 16, wherein the effective amount of CSF-1 at the site of administration is upheld for at least 1 day, preferably at least 4 days, especially at least 7 days, preferentially up to 14 days or longer.
